# FASCICULE DE BREVET EUROPEEN

(11) **EP 2 470 493 B1**
(45) Date de publication et mention de la délivrance du brevet: **25.06.2014**
(21) Numéro de dépôt: 10751875.5
(22) Date de dépôt: 23.08.2010
(51) Int. Cl.: C07C 67/08, C07C 69/14, C07C 67/54

(54) **PROCEDE D'ESTERIFICATION D'ACIDE CARBOXYLIQUE**
VERFAHREN ZUR VERESTERUNG VON CARBONSÄURE
PROCESS FOR ESTERIFICATION OF CARBOXYLIC ACID

(30) Priorité: 24.08.2009 FR 0955762
(43) Date de publication de la demande: 04.07.2012
(73) Titulaire: Rhodia Opérations, 93306 Aubervilliers (FR)
(72) Inventeur: AMOROS, Daniel, 69200 Venissieux (FR); PITIOT, Pascal, 69008 Lyon (FR); BREHELIN, Mathias, 69003 Lyon (FR); TRESMONDI, Alexandre, 13023-200 Campinas - SP (BR)
(74) Mandataire: Blanchard, Isabelle Jackie
(86) Numéro de dépôt international: PCT/EP2010/062265
(87) Numéro de publication internationale: WO 2011/023662

(56) Documents cités:
- EP-A1- 1 502 910
- EP-A1- 1 529 828
- EP-A2- 0 119 833
- WO-A1-98/25876
- DE-A1-102007 063 507
- US-A1- 2001 016 666
- US-A1- 2009 198 083

## Description

La présente invention concerne un procédé d'estérification à partir d'alcools et d'acides carboxyliques en catalyse hétérogène dans lequel la réaction d'estérification est conduite à de hautes températures permettant d'accroître le compromis de sélectivité, de conversion et de cinétique dans différents types de dispositifs de mise en oeuvre du procédé.

### ART ANTERIEUR

Il est connu de fabriquer des esters carboxyliques par réaction d'estérification d'un alcool et d'un acide carboxylique. Cette réaction d'estérification est aussi appelée réaction de Fisher. La réaction inverse est une hydrolyse. Les réactions dans les deux sens sont très lentes et donc peu performantes sans l'aide d'un catalyseur : un proton libre provenant soit d'un acide fort riche en protons libres en solution aqueuse, ou de l'eau dans laquelle l'acide carboxylique est en solution. Elle est aussi quasi athermique, c'est-à-dire qu'elle ne dégage ni absorbe de la chaleur. Ainsi une variation de la température n'a aucune influence sur le rendement comme il est connu de la loi expérimentale de Van Hoff. De même, une variation de la pression n'entraîne aucun déplacement de l'équilibre dans la mesure où les réactifs et les produits sont des liquides dans la plus part des cas, à teneur constante de réactants. Seule une augmentation de la température accélère la réaction et permet d'atteindre plus rapidement l'équilibre d'estérification.

La conversion de la réaction dépend très peu de la nature de l'acide carboxylique utilisé, mais dépend surtout de la classe de l'alcool utilisé. Pour des réactifs introduits en quantités équimolaires, elle est de 67 % avec un alcool primaire comme le méthanol de 60 % avec un alcool secondaire comme l'isopropanol, et de seulement 5% si l'alcool est un alcool tertiaire comme le tertbutanol.

Pour augmenter le rendement, il est connu d'utiliser un des réactif en excès, en général le moins cher, ce qui modifiera le taux d'avancement final de la réaction et donc le rendement. Ceci permet également de déplacer l'équilibre dans le sens direct d'estérification, notamment en distillant l'ester au fur et à mesure de sa formation, s'il est le plus volatil, ou en éliminer l'eau. Pour cela, on peut par exemple utiliser un entraînement avec un inerte, en ajoutant au système réactionnel un solvant relativement volatil et formant avec l'eau un hétéroazéotrope ou encore incorporer au mélange réactionnel une substance déshydratante. Cela pose toutefois des problèmes, car d'une part, même s'il est mis en excès, toute l'eau peut ne pas être consommée. D'autre part, il faut par la suite séparer l'ester de ce produit, ce qui peut entraîner des complications et une baisse du rendement.

Concernant la cinétique, la réaction d'estérification non catalysée est assez lente. La vitesse évolue aussi selon la classe des alcools : elle décroît quand on passe d'un alcool primaire à un alcool secondaire, puis à un alcool tertiaire. On peut augmenter la température de manière à améliorer grandement la cinétique selon la loi d'Arrhenius, et utiliser un catalyseur acide qui permet d'augmenter le caractère électrophile du groupe carboxyle.

La plupart des réactions d'estérification à l'échelle industrielle utilisent un catalyseur homogène acide, tel que l'acide sulfurique. On peut citer à cet effet la publication « Chemie Ingenieur Technik 43, 1971, N°18, 1001-1007 qui décrit un procédé de préparation d'esters à partir d'alcools et d'acides carboxyliques dans une colonne de distillation réactive en utilisant comme catalyseur homogène l'acide sulfurique. Mais ce composé est un puissant oxydant qui peut oxyder l'alcool ou même le déshydrater et provoquer des corrosions sur les installations.

Par ailleurs, au-delà du rendement et de la cinétique, il est nécessaire de conduire la réaction d'estérification avec si possible la meilleure sélectivité et un temps de séjour minimal. Toutefois, en catalyse homogène les températures élevées permettent que l'équilibre s'établisse dans un temps raisonnable pour favoriser le produit le plus stable, favorisant la cinétique, mais au détriment de la sélectivité.

La catalyse hétérogène a permis de s'affranchir des problèmes de corrosions des installations et de sélectivité de la réaction d'estérification, tout en simplifiant le procédé.

Il est connu d'utiliser des catalyseurs hétérogènes à des températures généralement comprises entre 50 et 100°C, car la stabilité de ces catalyseurs a de hautes températures n'est pas acquise.

Ainsi par exemple la demande WO2007/099071 décrit la fabrication d'esters, tels que l'ester levulinate d'éthyle, par distillation réactive homogène ou hétérogène en utilisant un corps extractif permettant d'entrainer en pieds de colonne l'ester produit. On peut notamment utiliser le cyclohexane à cet effet. Le catalyseur est préférentiellement une résine sulfonique. Le procédé met notamment en oeuvre le catalyseur à une température inférieure à 100°C et à pression atmosphérique.

Le brevet US60288215 décrit la fabrication d'esters, tels que l'ester acétate de butyle, à partir de butanol et d'acide acétique par distillation réactive hétérogène. Le catalyseur est de type résine sulfonique et l'ester produit sort en partie inférieure de la colonne réactive. Le procédé met en oeuvre le catalyseur à une température comprise entre 50 et 160°C et à une pression inférieure à 2 bar.

La demande EP1220829 décrit la fabrication de l'ester acétate d'éthyle à partir d'éthanol et d'acide acétique par distillation réactive hétérogène. L'ester formé est récupéré dans la partie supérieure de la colonne réactive et le catalyseur utilisé est de type résine sulfonique. Le procédé décrit met en oeuvre le catalyseur à la pression atmosphérique ; et le distillat sort de la colonne à une température aux alentours de 70°C.

Toutefois, les procédés décrits dans ces documents ne permettent pas d'obtenir un excellent optimum entre la conversion, la cinétique et la sélectivité de la réaction. Il existait ainsi dans le domaine de l'estérification un besoin de mettre au point un procédé permettant d'éviter les inconvénients cités précédemment et d'obtenir un tel compromis. Il existait par ailleurs une nécessité de diminuer les coûts de production de manière importante pour la conduite de tels procédés d'estérification.

### INVENTION

Il est apparu de manière tout a fait surprenante que l'utilisation de catalyseurs hétérogènes acides particuliers dans une réaction d'estérification mettant en oeuvre des alcools et des acides carboxyliques à des températures élevées permettait d'atteindre tous les objectifs mentionnés précédemment ; notamment d'éviter une corrosion des installations tout en permettant un excellent compromis entre la conversion, la cinétique et la sélectivité de la réaction.

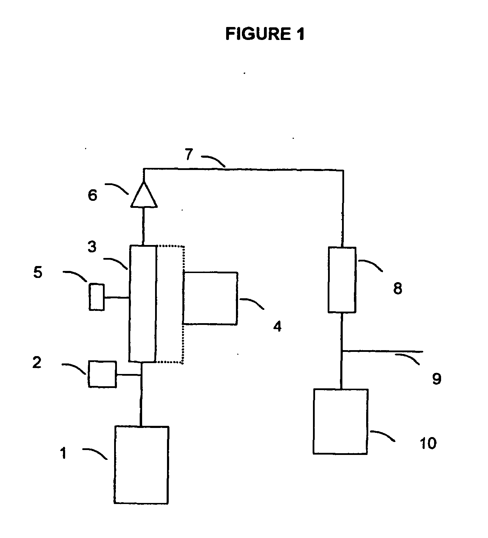

Ces catalyseurs particuliers sont identifiés selon la présente invention par leur haute stabilité thermique dans le temps lors de la réaction d'estérification à haute température. Ils sont caractérisés par le maintien du taux de conversion qu'ils sont capables d'apporter à cette réaction pendant la durée totale du test P décrit ci-après, notamment selon le dispositif de la Figure 1.

Il apparait par ailleurs que l'on obtient une excellente conversion et sélectivité de la réaction même en utilisant un rapport molaire acide carboxylique / alcool compris entre 0,5 et 2 ; c'est-à-dire sans procéder à un fort déséquilibre molaire des réactifs.

Il apparait au surplus que cette augmentation de température utilisée lors de la réaction d'estérification peut être valorisée dans l'installation pour diminuer les couts en énergie, notamment pour les étapes de purification ultérieures des réactants.

La conduite de la réaction a de plus hautes températures permet également de diminuer le volume nécessaire à la réaction ce qui se révèle être d'un grand avantage pour conduire la réaction dans des dispositifs de taille moindre et donc moins couteux. La conduite de la réaction a de plus hautes températures permet également de diminuer la quantité nécessaire de catalyseurs à la réaction, ce qui diminue les couts et le traitement des effluents.

### DESCRIPTION DETAILLEE DE L'INVENTION

La présente invention a ainsi pour premier objet un procédé de fabrication d'un ester carboxylique par réaction d'estérification d'un acide carboxylique et d'un alcool à une température comprise entre 100 et 200°C ; en présence d'un catalyseur d'estérification hétérogène acide ;
ledit catalyseur présentant un taux de conversion compris entre 20 % et 67 %, plus préférentiellement entre 50 et 67 %, pendant toute la durée du test P suivant : un mélange équimolaire d'acide acétique et d'éthanol est stocké à température ambiante dans un réservoir (1) disposé sur une balance. Il est transféré en mode continu par une pompe volumétrique (2) à un débit de 100 gr/h., mesuré par différence de pesée sur la balance du réservoir, vers un réacteur lit fixe (3) contenant environ 1 g de catalyseur sec à tester. Le diamètre intérieur du lit est de 4 mm et sa longueur de 220 mm. Le catalyseur est maintenu entre deux grilles. La température de la réaction est contrôlée par un bain thermostaté (4) contenant un caloporteur qui circule dans la double enveloppe située autour du réacteur. La température de la réaction est de 140°C et est contrôlée par une sonde thermique (5). Un clapet de contrepression (6) situé sur la ligne (7) en sortie du réacteur est réglé à une pression choisie pour maintenir le milieu réactionnel en phase liquide à la température de 140°C. Le mélange réactionnel est détendu à pression atmosphérique à la sortie du clapet. Il est condensé et refroidi à 25 °C par un échangeur (8) alimenté en eau réfrigérée. Une partie du mélange refroidi est échantillonné via un dispositif de prélèvement (9) pour analyse chromatographique, l'autre partie est récupérée dans le réservoir (10). L'activité du catalyseur à haute température est contrôlée par l'analyse de la stabilité de la conversion des réactifs pour une durée de 300 h de fonctionnement en mode continu. Un tel dispositif peut correspondre à celui de la Figure 1.

On peut alors éventuellement procéder à une ou plusieurs étapes de séparation des produits issus de la réaction d'estérification, de manière à isoler l'ester formé.

On peut éventuellement valoriser la haute température utilisée lors de la réaction d'estérification pour réduire ou valoriser la consommation d'énergie de l'ensemble du dispositif industriel, comprenant notamment au moins un moyen de conduire la réaction d'estérification et au moins un moyen de conduire la ou les étapes de séparation ultérieures des réactants. On entend notamment par réduction ou valorisation de l'énergie du dispositif industriel, l'utilisation des flux à haute température de l'étape réactionnelle d'estérification pour chauffer les flux à basse température utilisées pour les étapes de purification ultérieures.

En pratique, le flux à haute température sortant, directement ou non directement, du réacteur de la réaction d'estérification est généralement traité dans un échangeur thermique, tel que par exemple un condenseur, un pot de flash ou un rebouilleur, pour échanger son énergie avec des moyens permettant de conduire la ou les étapes de séparation ultérieures des réactants.

Le procédé de l'invention peut être conduit de diverses manières et dans différents types de réacteurs possibles. Il n'y a pas de limitation aux dispositifs pouvant mettre en oeuvre la réaction de l'invention. Des informations détaillées sur les réacteurs et dispositifs possibles sont données ci-dessous.

D'une manière générale, le procédé de l'invention comprend au moins les étapes suivantes :
- a) alimenter au moins un alcool et un acide carboxylique dans un réacteur ;
- b) faire réagir l'alcool et l'acide en présence d'un catalyseur d'estérification hétérogène acide ;
- c) procéder à une ou plusieurs étapes de séparation des produits issus de
la réaction de l'étape b) de manière à isoler l'ester formé,
ledit procédé étant caractérisé en ce que
- l'alcool est choisi dans le groupe comprenant : le méthanol, l'éthanol, le propanol et le butanol,
- l'acide carboxylique est l'acide acétique,
- ladite étape b) est opérée à une température comprise entre 130 et 170°C, une pression comprise entre 1 et 20 bar, ledit catalyseur d'estérification étant choisi parmi les résines sulfoniques Amberlyst 70 et Lewatit K2431.

Plusieurs procédés conventionnels de séparation peuvent être utilisés pour séparer les réactants, notamment l'ester carboxylique, des autres constituants. On peut citer par exemple comme procédé de séparation : la distillation, la condensation, la chromatographie, la séparation membranaire et l'extraction. Il est possible d'utiliser un seul de ces moyens de séparation, ou plusieurs de manière continu ou parallèle. On préfère notamment opérer une ou plusieurs distillations ou alors une condensation suivie d'une ou plusieurs distillations.

La pression de la réaction du procédé est préférentiellement comprise entre 1 et 20 bar, notamment entre 2,5 et 13 bar, plus préférentiellement encore entre 3 et 5 bar.

La réaction de l'invention est tout préférentiellement conduite à une température comprise entre 130 et 170°C et une pression comprise entre 3 et 13 bar.

L'alcool est choisi dans le groupe comprenant:
le méthanol, l'éthanol, le propanol et le butanol. On peut notamment utiliser lors de cette réaction des alcools primaires ou
secondaires. On préfère notamment les alcools de point d'ébullition bas comme le méthanol et l'éthanol. L'acide carboxylique est notamment l'acide acétique. L'ester préféré est l'éthyle acétate.

Dans le procédé de l'invention, le rapport molaire acide carboxylique / alcool peut être compris entre 1 et 10.

Les catalyseurs sont l'Amberlyst 70 de Rhom Haas et la Lewatit K2431 de Lanxess. Pour ce qui concerne le catalyseur Lewatit K2431 ont peut se référer à la Fiche Information Produit du 26/08/08 de Lanxess. Pour ce qui concerne le catalyseur Amberlyst 70 on peut se référer à la fiche « Amberlyst ^{™} 70 Strongly Acidic Catalyst » de Rhom Haas.

Pour un mélange équimolaire acide / alcool, la concentration en catalyseur est généralement comprise entre 1 et 10 % en poids en réacteur agité slurry, avec pour un lit fixe un temps de contact entre 10 secondes et 1 heure, et préférentiellement entre 10 secondes et 5 minutes à 140 °C.

La réaction d'estérification entre l'acide carboxylique et l'alcool peut, par exemple et sans aucune limitation, être effectuée dans des réacteurs conventionnels ou des colonnes de distillations réactives.

La réaction peut notamment fonctionner en discontinu, en continu ou en semi-continu.

L'alcool et l'acide peuvent être alimentés dans la zone de réaction de diverses manières. Par exemple en même temps par des moyens d'alimentation différents, ou alors en pré-mélange, ou encore de manière différée. On peut également utiliser de l'acide ou de l'alcool pur ou pratiquement pur ou encore des mélanges d'acide et d'alcool comprenant d'autres composés organiques ou inorganiques comme l'eau. L'acide et l'alcool peut notamment provenir d'un moyen de production ou de séparation antérieur, telle qu'une colonne de distillation.

Selon une première variante de l'invention, le procédé peut être conduit dans un réacteur conventionnel dans lequel les catalyseurs hétérogènes sont en suspension (slurry), immobilisé sur lit fixe, ou sur lit fluidisé.

Les catalyseurs peuvent être immobilisés à la surface de supports inertes telles que des grilles métalliques ou des éléments en silice qui forment une structure fixe, dite lit fixe, ou une structure mouvante, dite lit fluidisé, placé dans le réacteur.

On peut avoir notamment recours à la technique d'adsorption réactive dans le réacteur pour accroître la conversion. Un agent adsorbant permet par exemple de retirer l'eau formée pendant la réaction.

Lorsque le procédé est réalisé dans un réacteur conventionnel, le rapport molaire acide carboxylique / alcool est préférentiellement compris entre 5 et 10.

On préférera coupler au réacteur conventionnel d'estérification une colonne de distillation dite primaire pour séparer l'ester carboxylique des autres constituants de la réaction. On pourra également utiliser au surplus une ou plusieurs autres colonnes de distillation. On peut par exemple acheminer le flux à haute température de la colonne de distillation primaire vers un échangeur thermique de manière à fournir de la chaleur au pied de la ou des autres colonnes de distillation.

Selon une deuxième variante de l'invention, on peut notamment utiliser comme dispositif une colonne de distillation réactive, qui permet d'effectuer à la fois au moins une réaction chimique en présence de catalyseur et la séparation par distillation du mélange réactionnel obtenu. Le procédé et le dispositif de l'invention peuvent s'appliquer à diverses réactions équilibrées, en phase liquide, pour lesquelles on peut isoler le produit de réaction par distillation dans les conditions de température et de pression auxquelles on réalise la réaction.

La colonne de distillation réactive comprend notamment au moins une zone réactionnelle et au moins une zone non réactionnelle.

Le corps de garnissage éventuellement utilisé dans le procédé de la présente invention est choisi en fonction de l'efficacité nécessaire. Le corps de garnissage peut être choisi parmi les corps de garnissage bien connus de l'homme du métier, tels que par exemple des solides sous forme d'anneaux, d'extrudés polylobés ou de selles. A titre d'exemple non limitatif de corps de garnissage, on peut citer les anneaux de Raschig, les anneaux de Pall, les anneaux d'Intos, les selles de Berl, les selles Novalox et les selles Intalox. Mais le corps de garnissage peut aussi être choisi parmi les garnissages structurés, par exemple de type FLEXIPAC (marque déposée) commercialisés par la société Koch, ou SULZER CHEMTECH ou SULZER (marques déposées) commercialisés par la société Sulzer.

La zone réactionnelle de la colonne de distillation réactive comprend généralement au moins un lit catalytique d'estérification. Le catalyseur peut être enfermé dans au moins une enveloppe perméable, enveloppe constituée par exemple par une toile en tissu, en matériau synthétique par exemple en polypropylène, ou en tissu métallique. Le catalyseur peut également être disposé en vrac, c'est-à-dire librement, à l'intérieur de chaque lit catalytique de la zone catalytique. Dans ce cas, pour maintenir le catalyseur en place et pour éviter qu'il soit entraîné par le courant de liquide qui le traverse, on prévoit généralement que tout lit catalytique compris dans la zone catalytique repose sur tout dispositif permettant le passage du liquide mais imperméable aux particules catalytiques, telles que par exemple une grille de type Johnson ou des busettes de type Johnson réparties régulièrement sur une surface imperméable aux flux gazeux, liquides ou solides. Avantageusement, ledit dispositif est légèrement surélevé par rapport à l'arrivée du ou des moyens de circulation sensiblement radiale, sur le fond de la zone catalytique, de manière à créer une zone de répartition située en partie au-dessous du lit catalytique de la zone catalytique.

Lorsque le catalyseur est disposé en vrac, on peut prévoir de l'utiliser sous forme de lit fixe, de lit expansé ou de lit fluidisé. Que le catalyseur soit disposé en vrac ou enfermé dans au moins une enveloppe, le taux de vide que l'on peut lui conférer est compris généralement entre 30 et 70%.

Pour ce qui concerne la seconde variante utilisant une colonne de distillation réactive, le procédé comprend généralement au moins les étapes suivantes :
- a) alimenter au moins un alcool et un acide carboxylique dans une colonne de distillation comprenant au moins une zone réactionnelle et au moins une zone non réactionnelle ;
- b) faire réagir l'alcool et l'acide dans la ou les zones réactionnelle en présence d'un catalyseur hétérogène, et séparer par distillation les composés formés ;
- c) procéder à une ou plusieurs séparations des produits issus de la réaction de l'étape b) de manière à isoler l'ester formé ; et
- d) éventuellement valoriser la température utilisée pour la distillation réactive de l'étape b) dans la ou les séparations de l'étape c) de façon à réduire la consommation énergétique de cet ensemble.

L'alcool est préférentiellement alimenté dans la partie inférieure de chaque zone réactionnelle ou en dessous de chacune de ces zones. L'acide est tout préférentiellement alimenté dans la partie supérieure de chaque zone réactionnelle ou en dessus de chacune de ces zones.

On préfère tout particulièrement que la réaction à l'étape b) soit effectuée de manière à ce que le rapport molaire acide carboxylique / alcool soit compris entre 1 et 2.

Lorsque l'on utilise un alcool de bas point d'ébullition, tel que l'éthanol, on récupère notamment en tête de colonne de distillation réactive un mélange azéotrope ternaire comprenant de l'ester, de l'alcool et de l'eau ; et en pieds de colonne l'acide et l'eau. La concentration en éthanol dans ce mélange azéotrope est généralement comprise entre 0,5 et 1,5 % en poids, préférentiellement aux alentours de 0,9 % en poids. On préfère notamment la réaction de l'acide acétique avec de l'éthanol, conduisant à une récupération de l'ester carboxylique, de l'eau et de l'alcool n'ayant pas réagi en tête de colonne, et l'acide carboxylique en pied de colonne.

La proportion en catalyseur hétérogène dans les sections réactives est préférentiellement comprise entre 10 et 50 % en volume.

On préférera coupler à la colonne de distillation réactive au moins une colonne de distillation pour séparer l'ester carboxylique des autres constituants de la réaction. On peut par exemple acheminer le flux à haute température de l'étape réactionnel d'estérification de la colonne de distillation réactive vers un échangeur thermique de manière à fournir de la chaleur au pied de cette colonne de distillation.

On pourra avantageusement combiner les deux variantes de l'invention mentionnés précédemment ; c'est-à-dire une réaction d'estérification dans un réacteur conventionnel, notamment un réacteur lit fixe, et une réaction d'estérification dans une colonne de distillation réactive ; par exemple soit de façon consécutive, soit de façon parallèle. Le mode parallèle est préférable pour recycler un des flux sortant la colonne de distillation réactive vers le réacteur conventionnel. On peut éventuellement utiliser le flux à haute température de la colonne de distillation réactive pour réduire la consommation énergétique des étapes de séparations ultérieures des réactants. On peut notamment utiliser le flux à haute température de la colonne de distillation réactive et de la colonne de distillation primaire consécutive au réacteur conventionnel pour réduire la consommation énergétique des étapes de séparations ultérieures des réactants.

Un langage spécifique est utilisé dans la description de manière à faciliter la compréhension du principe de l'invention. Il doit néanmoins être compris qu'aucune limitation de la portée de l'invention n'est envisagée par l'utilisation de ce langage spécifique. Des modifications, améliorations et perfectionnements peuvent notamment être envisagés par une personne au fait du domaine technique concerné sur la base de ses propres connaissances générales.

Le terme et/ou inclut les significations et, ou, ainsi que toutes les autres combinaisons possibles des éléments connectés à ce terme.

D'autres détails ou avantages de l'invention apparaîtront plus clairement au vu des exemples donnés ci-dessous uniquement à titre indicatif.

### PARTIE EXPERIMENTALE

### Exemple 1 : Réacteur simple

L'acétate d'éthyle (RN-CAS 141-78-6) est classiquement synthétisé par estérification de l'acide acétique (RN-CAS 64-19-7) avec l'éthanol (RN-CAS 64-17-5). Cette réaction athermique et équilibrée est accélérée par une catalyse acide hétérogène avec des catalyseurs à potentiel acide comme les résines sulfoniques.

Deux résines sont utilisées dans cette partie expérimentale :
- La résine A70 commercialisée par Rohm&Haas (taux de conversion de 67 % selon le test P)
- La résine K2431 commercialisée par Lanxess (taux de conversion de 67 % selon le test P)

Après 4 lavages à l'eau déminéralisée avec 2 volumes d'eau pour 1 volume de résine humide, puis 4 lavages à l'éthanol absolu avec 2 volumes d'alcool pour 1 volume de résine humide, chacune de ces 2 résines peut être engagée en réaction chimique.

Le tableau 1 ci-dessous montre l'évolution cinétique de la réaction dans des conditions équimolaires acide-alcool avec 5% massique de résine A70 en réacteur agité sous pression 12 bar ; à respectivement 70 et 160°C. Pour chaque résine, on estime à 50 kJ.mol⁻¹ l'énergie d'activation des réactions directe et inverse.

**Table 1**

| **Temps de réaction (min)** | **Masse d'acétate d'éthyle à 70°C (g)** | **Masse d'acétate d'éthyle à 160°C (g)** |
|---|---|---|
| 0 | 0 | 0 |
| 10 | 20 | 60 |
| 20 | 32 | 60 |
| 30 | 40 | 60 |
| 40 | 45 | 60 |
| 50 | 50 | 60 |
| 60 | 51 | 60 |

Le tableau 2 ci-dessous montre l'évolution cinétique de la réaction dans des conditions équimolaires acide-alcool avec 5% massique de résine K2431 en réacteur agité sous pression 12 bar ; à respectivement 70 et 160°C. Pour chaque résine, on estime à 50 kJ.mol⁻¹ l'énergie d'activation des réactions directe et inverse.

**Table 2**

| **Temps de réaction (min)** | **Masse d'acétate d'éthyle à 70°C (g)** | **Masse d'acétate d'éthyle à 160°C (g)** |
|---|---|---|
| 0 | 0 | 0 |
| 10 | 25 | 60 |
| 20 | 37 | 60 |
| 30 | 45 | 60 |
| 40 | 50 | 60 |
| 50 | 52 | 60 |
| 60 | 54 | 60 |

Sur les chromatogrammes obtenus par analyse CPG classique avec une dilution volumique du brut réactionnel d'un facteur 200, on n'observe pas de chimie parasite majoritaire, malgré le niveau thermique élevé.

## Revendications

1. Procédé de fabrication d'un ester carboxylique par réaction d'estérification d'un acide carboxylique et d'un alcool comprenant au moins les étapes suivantes :
• a) alimenter au moins un alcool et un acide carboxylique dans un réacteur ;
• b) faire réagir l'alcool et l'acide en présence d'un catalyseur d'estérification. hétérogène acide ;
• c) procéder à une ou plusieurs étapes de séparation des produits issus de la réaction de l'étape b) de manière à isoler l'ester formé,
ledit procédé étant **caractérisé en ce que**
- l'alcool est choisi dans le groupe comprenant : le méthanol, l'éthanol, le propanol et le butanol,
- l'acide carboxylique est l'acide acétique,
- ladite étape b) est opérée à une température comprise entre 130 et 170°C, une pression comprise entre 1 et 20 bar, ledit catalyseur d'estérification étant choisi parmi les résines sulfoniques Amberlyst 70 et Lewatit K2431.

2. Procédé selon la revendication 1, **caractérisé en ce qu'**il comprend au moins les étapes suivantes :
• a) alimenter au moins un alcool et un acide carboxylique dans un réacteur ;
• b) faire réagir l'alcool et l'acide en présence d'un catalyseur hétérogène ;
• c) procéder à une ou plusieurs étapes de séparation des produits issus de la réaction de l'étape b) de manière à isoler l'ester formé ; et
• d) valoriser la température utilisée lors de l'étape b) pour la ou les étapes de séparation de l'étape c), de façon à réduire la consommation énergétique de cet ensemble.

3. Procédé selon la revendication 1 ou la revendication 2, **caractérisé en ce que** la réaction d'estérification est conduite à une température comprise entre 130 et 170 °C et une pression comprise entre 3 et 13 bar.

4. Procédé selon l'une quelconque des revendications précédentes, **caractérisé en ce que** l'ester formé est l'éthyle acétate.

5. Procédé selon l'une quelconque des revendications précédentes, **caractérisé en ce que** le rapport molaire acide carboxylique / alcool utilisé dans la réaction d'estérification est compris entre 1 et 10.

6. Procédé selon l'une quelconque des revendications précédentes, **caractérisé en ce que** la réaction d'estérification est conduite dans un réacteur conventionnel présentant des catalyseurs en suspension, immobilisé sur lit fixe, ou sur lit fluidisé.

7. Procédé selon l'une quelconque des revendications 1 à 5, **caractérisé en ce que** la réaction d'estérification est conduite dans une colonne de distillation réactive.

8. Procédé selon la revendication 7 comprenant au moins les étapes suivantes :
• a) alimenter au moins un alcool et un acide carboxylique dans une colonne de distillation comprenant au moins une zone réactionnelle et au moins une zone non réactionnelle ;
• b) faire réagir l'alcool et l'acide dans la ou les zones réactionnelle en présence d'un catalyseur hétérogène, et séparer par distillation les composés formés ;
• c) procéder à une ou plusieurs séparations des produits issus de la réaction de l'étape b) de manière à isoler l'ester formé ; et
• d) éventuellement valoriser la température utilisée pour la distillation réactive de l'étape b) dans la ou les séparations de l'étape c) de façon à réduire la consommation énergétique de cet ensemble.

9. Procédé selon la revendication 7 ou 8, **caractérisé en ce que** le rapport molaire acide carboxylique / alcool utilisé dans la réaction est compris entre 1 et 2.

10. Procédé selon l'une quelconque des revendications 7 à 9 **caractérisé en ce qu'**il concerne la réaction de l'acide acétique avec de l'éthanol, conduisant à une récupération de l'ester carboxylique, de l'eau et de l'alcool n'ayant pas réagi en tête de colonne, et l'acide carboxylique en pied de colonne.

11. Procédé selon l'une quelconque des revendications précédentes, **caractérisé en ce que** l'on procède à une réaction d'estérification dans un réacteur conventionnel et une réaction d'estérification dans une colonne de distillation réactive ; soit de façon consécutive, soit de façon parallèle.

## Patentansprüche

1. Verfahren zur Herstellung eines Carbonsäureesters durch Veresterungsreaktion einer Carbonsäure und eines Alkohols, das mindestens folgende Schritte umfasst:
• a) Einspeisen mindestens eines Alkohols und einer Carbonsäure in einen Reaktor;
• b) Umsetzen des Alkohols und der Säure in Gegenwart eines sauren heterogenen Veresterungskatalysators;
• c) Durchführen eines oder mehrerer Schritte zur Trennung der Produkte aus der Umsetzung von Schritt b) zur Isolierung des gebildeten Esters,
wobei das Verfahren **dadurch gekennzeichnet ist, dass**
- man den Alkohol aus der Gruppe umfassend Methanol, Ethanol, Propanol und Butanol auswählt,
- es sich bei der Carbonsäure um Essigsäure handelt,
- der Schritt b) bei einer Temperatur zwischen 130 und 170°C und einem Druck zwischen 1 und 20 bar betrieben wird, wobei der Veresterungskatalysator aus den Sulfonsäureharzen Amberlyst 70 und Lewatit K2431 ausgewählt wird.

2. Verfahren nach Anspruch 1, **dadurch gekennzeichnet, dass** es mindestens folgende Schritte umfasst:
• a) Einspeisen mindestens eines Alkohols und einer Carbonsäure in einen Reaktor;
• b) Umsetzen des Alkohols und der Säure in Gegenwart eines heterogenen Katalysators;
• c) Durchführen eines oder mehrerer Schritte zur Trennung der Produkte aus der Umsetzung von Schritt b) zur Isolierung des gebildeten Esters und
• d) Nutzen der bei Schritt b) verwendeten Temperatur für den bzw. die Trennschritte von Schritt c) zur Verringerung des Energie-verbrauchs dieses Aufbaus.

3. Verfahren nach Anspruch 1 oder Anspruch 2, **dadurch gekennzeichnet, dass** die Veresterungsreaktion bei einer Temperatur zwischen 130 und 170°C und einem Druck zwischen 3 und 13 bar durchgeführt wird.

4. Verfahren nach einem der vorhergehenden Ansprüche, **dadurch gekennzeichnet, dass** es sich bei dem gebildeten Ester um Essigsäureethylester handelt.

5. Verfahren nach einem der vorhergehenden Ansprüche, **dadurch gekennzeichnet, dass** das bei der Veresterungsreaktion verwendete Carbonsäure/Alkohol-Molverhältnis zwischen 1 und 10 liegt.

6. Verfahren nach einem der vorhergehenden Ansprüche, **dadurch gekennzeichnet, dass** die Veresterungsreaktion in einem herkömmlichen Reaktor mit suspendierten oder an einem Festbett oder an einer Wirbelschicht immobilisierten Katalysatoren durchgeführt wird.

7. Verfahren nach einem der Ansprüche 1 bis 5, **dadurch gekennzeichnet, dass** die Veresterungsreaktion in einer Reaktivdestillationskolonne durchgeführt wird.

8. Verfahren nach Anspruch 7, das mindestens folgende Schritte umfasst:
• a) Einspeisen mindestens eines Alkohols und einer Carbonsäure in eine Destillationskolonne mit mindestens einer Reaktionszone und mindestens einer Nichtreaktionszone;
• b) Umsetzen des Alkohols und der Säure in der Reaktionszone bzw. den Reaktionszonen in Gegenwart eines heterogenen Katalysators und destillative Trennung der gebildeten Verbindungen;
• c) Durchführen einer oder mehrerer Trennungen der Produkte aus der Umsetzung von Schritt b) zur Isolierung des gebildeten Esters und
• d) gegebenenfalls Nutzen der für die Reaktivdestillation von Schritt b) verwendeten Temperatur bei der Trennung bzw. den Trennungen von Schritt c) zur Verringerung des Energieverbrauchs dieses Aufbaus.

9. Verfahren nach Anspruch 7 oder 8, **dadurch gekennzeichnet, dass** das bei der Reaktion verwendete Carbonsäure/Alkohol-Molverhältnis zwischen 1 und 2 liegt.

10. Verfahren nach einem der Ansprüche 7 bis 9, **dadurch gekennzeichnet, dass** es die Umsetzung von Essigsäure mit Ethanol betrifft, was zur Gewinnung des Carbonsäureesters, des Wassers und des nicht umgesetzten Alkohols am Kopf der Kolonne und der Carbonsäure am Sumpf der Kolonne führt.

11. Verfahren nach einem der vorhergehenden Ansprüche, **dadurch gekennzeichnet, dass** man entweder nacheinander oder parallel eine Veresterungsreaktion in einem herkömmlichen Reaktor und eine Veresterungsreaktion in einer Reaktivdestillationskolonne durchführt.

## Claims

1. Process for the manufacture of a carboxylic ester by an esterification reaction of a carboxylic acid and an alcohol comprising at least the following stages:
• a) feeding at least one alcohol and one carboxylic acid to a reactor;
• b) reacting the alcohol and the acid in the presence of an acidic heterogeneous esterification catalyst;
• c) carrying out one or more stages of separation of the products resulting from the reaction of stage b), so as to isolate the ester formed,
said process being **characterized in that**:
- the alcohol is chosen from the group consisting of methanol, ethanol, propanol and butanol,
- the carboxylic acid is acetic acid,
- said stage b) is carried out at a temperature of between 130 and 170°C and a pressure of between 1 and 20 bar, said esterification catalyst being chosen from the sulfonic resins Amberlyst 70 and Lewatit K2431.

2. Process according to Claim 1, **characterized in that** it comprises at least the following stages:
• a) feeding at least one alcohol and one carboxylic acid to a reactor;
• b) reacting the alcohol and the acid in the presence of a heterogeneous catalyst;
• c) carrying out one or more stages of separation of the products resulting from the reaction of stage b), so as to isolate the ester formed; and
• d) benefiting economically from the temperature used during stage b) for the separation stage or stages of step c), so as to reduce the energy consumption of this setup.

3. Process according to Claim 1 or Claim 2, **characterized in that** the esterification reaction is carried out at a temperature of between 130 and 170°C and a pressure of between 3 and 13 bar.

4. Process according to any one of the preceding claims, **characterized in that** the ester formed is ethyl acetate.

5. Process according to any one of the preceding claims, **characterized in that** the carboxylic acid/alcohol molar ratio used in the esterification reaction is between 1 and 10.

6. Process according to any one of the preceding claims, **characterized in that** the esterification reaction is carried out in a conventional reactor exhibiting catalysts in suspension, immobilized on a fixed bed, or on a fluidized bed.

7. Process according to any one of Claims 1 to 5, **characterized in that** the esterification reaction is carried out in a reactive distillation column.

8. Process according to Claim 7, comprising at least the following stages:
• a) feeding at least one alcohol and one carboxylic acid to a distillation column comprising at least one reaction region and at least one nonreaction region;
• b) reacting the alcohol and the acid in the reaction region or regions in the presence of a heterogeneous catalyst and separating, by distillation, the compounds formed;
• c) carrying out one or more separations of the products resulting from the reaction of stage b), so as to isolate the ester formed; and
• d) optionally benefiting economically from the temperature used for the reactive distillation of stage b) in the separation or separations of stage c), so as to reduce the energy consumption of this setup.

9. Process according to Claim 7 or 8, **characterized in that** the carboxylic acid/alcohol molar ratio used in the reaction is between 1 and 2.

10. Process according to any one of Claims 7 to 9, **characterized in that** it relates to the reaction of acetic acid with ethanol, resulting in the recovery of the carboxylic ester, the water and the unreacted alcohol at the column top and the carboxylic acid at the column bottom.

11. Process according to any one of the preceding claims, **characterized in that** an esterification reaction is carried out in a conventional reactor and an esterification reaction is carried out in a reactive distillation column, either consecutively or in parallel.
